# EUROPÄISCHE PATENTSCHRIFT

(11) **EP 2 173 388 B1**
(45) Veröffentlichungstag und Bekanntmachung des Hinweises auf die Patenterteilung: **22.02.2012**
(21) Anmeldenummer: 08775200.2
(22) Anmeldetag: 18.07.2008
(51) Int. Cl.: A61L 2/20, A61L 2/26, B01B 1/00, B01D 1/00

(54) **VERDAMPFER ZUM STERILISIEREN VON KUNSTSTOFFBEHÄLTERN**
VAPORIZER FOR STERILIZATION OF PLASTIC CONTAINERS
ÉVAPORATEUR POUR LA STÉRILISATION DE RÉCIPIENTS EN MATIÈRE PLASTIQUE

(30) Priorität: 23.07.2007 DE 102007034205
(43) Veröffentlichungstag der Anmeldung: 14.04.2010
(60) Teilanmeldung: 11192226.6
(73) Patentinhaber: Krones AG, 93073 Neutraubling (DE)
(72) Erfinder: BURGMEIER, Berthold, 89561 Dischingen/Eglingen (DE); ENGELHARD, Patrick, 84094 Elsendorf (DE); KNOTT, Josef, 84069 Schierling (DE)
(74) Vertreter: Bittner, Bernhard
(86) Internationale Anmeldenummer: PCT/EP2008/059421
(87) Internationale Veröffentlichungsnummer: WO 2009/013226

(56) Entgegenhaltungen:
- EP-A- 0 243 003
- WO-A-00/33967
- WO-A-03/006075
- DE-A1- 10 019 047
- US-A- 4 631 173
- US-A- 5 879 648

## Beschreibung

Die vorliegende Erfindung bezieht sich auf einen Verdampfer zum Sterilisieren von Behältnissen und insbesondere von Kunststoffbehältnissen. Derartige Verdampfer sind aus dem Stand der Technik bekannt. Dabei wird üblicherweise Wasserstoffperoxid (H₂O₂) innerhalb des Verdampfers verdampft und gemeinsam mit einem zugeführten Luftstrom in ein zu sterilisierendes Behältnis geführt. Durch dieses Einführen des Gemisches in das Behältnis kann dessen Innenwandung gereinigt bzw. sterilisiert werden.

Bei derartigen Verdampfern ist es jedoch erforderlich, die Menge des jeweils eingespritzen oder dem Behältnis zuzuführenden H₂O₂ präzise zu regulieren und dafür Sorge zu tragen, dass pro Behältnis jeweils die gleiche Menge an H₂O₂ zugeführt wird.

In der WO 00/33697 A ist eine Vorrichtung zum Erzeugen eine Aerosols aus einem gasförmigen Bestandteil, wie Sterilluft, und einem flüssigen Bestandteil, wie Peroxid, beschrieben, wobei das Peroxid in einem Zerstäuberbehälter fortlaufend zerstäubt wird und dem passierenden Gasstrom eingemischt wird. Dazu befördert eine Zerstäuberdüse das Peroxid in einen ringförmigen, gebündelten, aufwärtsgerichteten Luftstrom, der durch die Ringdüse gebildet wird. Durch eine Austrittsöffnung wird das Aerosol beispielsweise zu einer Flaschensterilisationsstation transportiert, wobei es auf seinem Weg noch mittels eines Heizaggregates erhitzt werden kann. Jedoch wird das Peroxid nicht in Form eines Verdampfungsprozesses verdampft, sondern als unbehandelte und nur lediglich leicht erhitze Lösung auf die Behältnisse aufgebracht.

Die WO 03/006075 beschreibt eine Vorrichtung sowie ein Verfahren, um eine hohe Verdampfungseffizienz von Luft und flüssigem Wasserstoffperoxid zu ermöglichen, wobei durch vier Öffnungen jeweils warme trockene Luft und durch eine Düse Wasserstoffperoxid in ein Gehäuse gesprüht wird, die sich dort vermischen und als Wasserstoff-Peroxid-Luft-Mischung durch eine Austrittsöffnung wieder aus dem Gehäuse abgeführt werden. Jedoch wird eine Verdampfung des Gemisches mittels des entsprechend auf ein Heizelement aufgebrachten Gemisches nicht bewirkt, so dass die entstandene Wasserstoff-Peroxid-Luft-Mischung weiterhin eine große Anzahl an entsprechend ungesättigten Luftbereichen in der Mischung aufweist.

In der DE 100 19 047 A1 ist eine Vorrichtung zum Sterilisieren von Verpackungen offenbart, welche ein Hauptrohr zur Zuführung von Sterilluft sowie zwei Vernebelungsdüsen aufweist. Während die eine Vernebelungsdüse ein erstes Gemisch aus Desinfektionsmittel und Wasser in das Hauptrohr einspritzt, wird von der zweiten Düse ein zweites Gemisch, welches aus einem oberflächenaktiven Stoff und Wasser besteht in das Hauptrohr eingespritzt. Beide Düsen münden innerhalb des Hauptrohres im Bereich eines Wärmetauschers, der das Gemisch aus dem ersten und zweiten Gemisch verdampft, welches mittels der Sterilluft zu einem Austritt des Hauptrohres, welcher mit einem Aseptiksystem in Verbindung steht, transportiert wird. Demzufolge benötigt die Vorrichtung einen enormen Wärmegrad, um die eingespritzte Mischung im Wesentlichen vollständig verdampfen zu können, wobei ansonsten Restmengen der zu verdampfenden Mischung im Bereich des Wärmetauschers verbleiben und diesen dort nachhaltig beschädigen könnten.

Aus der WO 2007/003313 sind ein Verfahren und eine Vorrichtung zum Überwachen eines Verdampfers bekannt. Bei diesem Verfahren wird auf einen erwärmten Heizkörper eine Flüssigkeit aufgebracht und verdampft und weiterhin ist seitlich an diesen Verdampfer eine Rohrleitung vorgesehen, durch welche hindurch erwärmte und sterile Luft von der Seite her in den Verdampfer bzw. dessen Gehäuse eingeleitet wird. Damit wird die Luftströmung hier senkrecht zu der Einspritzrichtung des Wasserstoffperoxids geführt. Diese Vorrichtung erlaubt eine effektive Zuführung von verdampften Wasserstoffperoxid in das Behältnis. Durch die Art der Zuführung ist jedoch eine exakte Regulierung der genauen Menge an Wasserstoffperoxid nur schwer möglich.

Die DE 100 40 861 A1 beschreibt eine Vorrichtung zum Sterilisieren von Packungen mit Wasserstoffperoxid. Dabei wird das Wasserstoffperoxid in Längsrichtung an einer warmen Oberfläche vorbeigeführt. Auf diese Weise wird das mit dem Wasserstoffperoxid in nebelform versetzte Trägergas schon an dieser warmen Oberfläche verdampft. Auch bei dieser Vorrichtung ist eine genaue Regulierung der zugeführten Menge an Wasserstoffperoxid nur schwer möglich.

Aus der EP 0 991 434 B1 ist ein multiples Flammpunkt-Verdampfungssystem bekannt. Auch wird in einen Verdampfer sowohl Wasserstoffperoxid als auch ein Trägergas eingeführt, wobei das Trägergas hier ebenfalls quer bezüglich der Einspritzrichtung des Wasserstoffperoxids fließt.

Die US 5,879,648 beschreibt eine Vorrichtung zum Desinfizieren von Behältern. Dabei wird in einen Verdampfer sowohl Wasserstoffperoxid als auch über parallel verlaufende Öffnungen ein Gas eingeführt. Auch bei dieser Vorrichtung ist eine Feinregulierung des aus dem Verdampfer in die Behältnisse gelangenden Gemisches nur schwer möglich.

Der vorliegenden Erfindung liegt daher die Aufgabe zugrunde, einen Verdampfer zu schaffen, der eine sehr genaue Regulierung des aus diesem austretenden Wasserstoffperoxid-Gasgemisches erlaubt.

Dies wird erfindungsgemäß durch einen Verdampfer nach Anspruch 1 und eine Verfahren Sterilisieren von Behältnissen nach Anspruch 13 erreicht. Vorteilhafte

Ausführungsformen und Weiterbildungen sind Gegenstand der abhängigen Ansprüche.

Der erfindungsgemäße Verdampfer zum Sterilisieren von Behältnissen weist ein Gehäuse auf, und eine erste in dem Gehäuse angeordnete Öffnung, um dem Gehäuse ein gasförmiges Medium zuzuführen. Weiterhin ist eine Einspritzeinrichtung vorgesehen, um in das Gehäuse eine Substanz einzuspritzen, welche Wasserstoffperoxid enthält. Weiterhin ist innerhalb des Gehäuses eine Heizeinrichtung angeordnet, um das Wasserstoffperoxid zu verdampfen. Daneben ist eine zweite in dem Gehäuse angeordnete Öffnung vorgesehen, um ein Gemisch aus dem gasförmigen Medium und dem verdampften Wasserstoffperoxid aus dem Gehäuse abzuführen. Erfindungsgemäß erstreckt sich eine Längsrichtung der Einspritzeinrichtung senkrecht zu einer Ebene der Heizeinrichtung, auf welcher das Wasserstoffperoxid auftrifft, und an die erste Öffnung schließt sich ein Rohrabschnitt an, der sich wenigstens abschnittsweise in einer Längsrichtung der Einspritzeinrichtung erstreckt, wobei die Einspritzeinrichtung wenigstens abschnittsweise innerhalb dieses Rohrabschnitts verläuft.

Bei einer weiteren möglichen Ausführungsform erstreckt sich eine Längsrichtung der Einspritzeinrichtung senkrecht zu einer Ebene der Heizeinrichtung und an die erste Öffnung schließt sich ein Rohrabschnitt an, der sich wenigstens abschnittsweise in einer Längsrichtung der Einspritzeinrichtung erstreckt, wobei sich der Rohrabschnitt wenigstens abschnittsweise innerhalb des Gehäuses erstreckt.

Bei der Einspritzeinrichtung handelt es sich bevorzugt um eine lanzenförmige Einspritzeinrichtung und die Längsrichtung der Einspritzeinrichtung ist durch die Längsrichtung dieser Lanze definiert. Bevorzugt handelt es sich bei der eingespritzten Substanz um Wasserstoffperoxid. Dieses kann dabei sowohl in flüssiger Form als auch in nebelartiger Konsistenz eingespritzt werden. Es wären jedoch auch andere als lanzenförmige Einspritzeinrichtungen denkbar. Bei anders gestalteten Einspritzeinrichtungen ist deren Längsrichtung auch durch die Strömungsrichtung des H₂O₂ beim Austritt durch eine Düse der Einspritzeinrichtung definiert.

Unter der Ebene der Heizeinrichtung wird beispielsweise die Ebene einer Heizplatte verstanden, auf welche das Wasserstoffperoxid auftrifft. Unter einer Erstreckung der Längsrichtung der Einspritzeinrichtung senkrecht zu dieser Ebene wird verstanden, dass der Winkel zwischen dieser Ebene und der Längsrichtung größer als 75°, bevorzugt größer 80° und besonders bevorzugt größer als 85° ist. Auf diese Weise wird erreicht, dass das aus der Einspritzeinrichtung austretende Wasserstoffperoxid wenigstens teilweise auch im Wesentlichen in senkrechter Richtung auf die Heizeinrichtung auftrifft. Damit wird bewirkt, dass auf der Heizeinrichtung eine gleichmäßige Verdampfung des Wasserstoffperoxids erfolgt. Durch die Anordnung der Einspritzeinrichtung innerhalb des Rohrabschnitts kann weiterhin erreicht werden, dass das aus dem Rohrabschnitt austretende Gas im Wesentlichen gleichförmig die Einspritzeinrichtung umgibt. Auf diese Weise kann ebenfalls eine gleichmäßige Anreicherung des Gases mit dem Wasserstoffperoxid bewirkt werden. Bei einer vorteilhaften Ausführungsform erstreckt sich der Rohrabschnitt innerhalb des Gehäuses wenigstens abschnittsweise in der Längsrichtung der Einspritzeinrichtung. Dies bedeutet, dass sich der Rohrabschnitt ausgehend von der ersten Öffnung nach innen und in der Längsrichtung der Einspritzeinrichtung erstreckt. Weiterhin weist bevorzugt der Rohrabschnitt eine erste Austrittsöffnung auf, die derart angeordnet ist, dass das gasförmige Medium in einer zu der Längsrichtung parallelen Richtung aus dem Rohrabschnitt austritt. Dies bedeutet, dass es sich bei demjenigem Rohrabschnitt, der parallel zu der Längsrichtung der Einspritzrichtung ist, um einen Endabschnitt dieses Rohrabschnitts handelt.

Bei einer weiteren vorteilhaften Ausführungsform weist der Rohrabschnitt eine zweite Austrittsöffnung auf, die zwischen der in dem Gehäuse angeordneten Öffnung (des Gehäuses) und der ersten Austrittsöffnung vorgesehen ist. Auf diese Weise kann ein Teilstrom auch in einer anderen Richtung als der Längsrichtung der Einspritzeinrichtung ausgeführt werden.

Vorzugsweise ist diese zweite Austrittsöffnung derart angeordnet, dass das durch diese Austrittsöffnung austretende gasförmige Medium unmittelbar in Richtung der zweiten Öffnung (des Gehäuses) strömt. Unter unmittelbar wird dabei verstanden, dass die Strömung ohne umgelenkt zu werden, in Richtung der zweiten Öffnung strömt. Vorzugsweise weist das Gehäuse abgesehen von der ersten Öffnung und der zweiten Öffnung keine Öffnung auf, durch welche hindurch ein gasförmiges Medium in das Gehäuse hinein oder aus dem Gehäuse heraustreten kann.

Vorzugsweise sind die erste Öffnung in dem Gehäuse und die zweite Öffnung in dem Gehäuse auf gegenüberliegenden Seiten beispielsweise auf gegenüberliegenden Seiten in der Ummantelung des Gehäuses oder auch auf gegenüberliegenden Seiten in Längsrichtung der Einspritzeinrichtung angeordnet. Vorteilhaft ist die erste Öffnung in einer Umfangswand des Gehäuse angeordnet. Bei einer weiteren vorteilhaften Ausführungsform weist der Rohrabschnitt einen gekrümmten Bereich auf, wobei dieser gekrümmte Bereich in einer Ausführungsform im Inneren des Gehäuses verläuft.

Bevorzugt ist bei dieser Ausführungsform die zweite Austrittsöffnung des Rohrabschnitts in dem gekrümmten Bereich desselben angeordnet.

Bei einer weiteren vorteilhaften Ausführungsform weist die Einspritzeinrichtung eine Austrittsdüse für das Wasserstoffperoxid auf und diese Austrittsdüse ist unterhalb der ersten Austrittsöffnung des Rohrabschnitts angeordnet. Dies bedeutet, dass der Rohrabschnitt und die Austrittsdüse nicht auf gleiche Höhe abschließen sondern die Austrittsdüse näher an der Heizeinrichtung ist als die Austrittsöffnung des Rohrabschnitts. Bei einer weiteren Ausführungsform sind an dem Rohrabschnitt Luftführungseinrichtungen vorgesehen, welche einen Teil der Luftströmung an eine Innenwandung des Verdampfers richten. Bevorzugt weist der Rohrabschnitt ein Innenrohr auf, welches die Einspritzeinrichtung konzentrisch umgibt und in wenigstens einem Bereich dieses Innenrohrs ist ein Außenrohr vorgesehen, wobei zwischen diesem Außenrohr und dem Innenrohr die Luftführungseinrichtungen vorgesehen sind. In diesem Falle handelt es sich bei dem Innenrohr um den erfindungsgemäßen Rohrabschnitt.

Bei einer weiteren vorteilhaften Ausführungsform sind die Einspritzeinrichtung und der Rohrabschnitt wenigstens abschnittsweise im Wesentlichen konzentrisch zueinander. Weiterhin ist besonders bevorzugt der Verdampfer bzw. dessen Gehäuse im Wesentlichen rotationssymetrisch bezüglich einer Zentralachse ausgebildet.

Bei einer weiteren vorteilhaften Ausführungsform ist die zweite Öffnung des Gehäuses in einem Bodenabschnitt des Gehäuses angeordnet. Bei dieser Ausführungsform wird besonders bevorzugt von oben in das Gehäuse das Wasserstoffperoxid und das gasförmige Medium eingeführt und nach unten hin das heißt durch den Bodenabschnitt abgezogen. Die Heizeinrichtung befindet sich bei dieser Ausführungsform zwischen dem Deckelabschnitt und dem Bodenabschnitt. Jedoch kann das entstehende Gemisch auch seitlich abgeführt werden.

Bei einer weiteren vorteilhaften Ausführungsform ist die Einspritzeinrichtung drehbar gegenüber dem Gehäuse angeordnet. Genauer gesagt ist die Einspritzeinrichtung und bevorzugt auch der Rohrabschnitt stationär und das Gehäuse dreht sich diesem gegenüber. Es wäre jedoch auch möglich, die Einspritzeinrichtung fest an dem Gehäuse und dieses stationär anzuordnen und gegenüber dem Gehäuse einen Auslaßstutzen zum Abführen des Gemisches drehbar anzuordnen.

Die vorliegende Erfindung ist weiterhin auf eine Anordnung zum Sterilisieren von Behältnissen und insbesondere von Kunststoffbehältnissen gerichtet, die wenigstens einen Verdampfer der oben beschriebenen Art aufweist. Bevorzugt weist diese Anordnung eine Erwärmungseinrichtung zum Erwärmen des gasförmigen Mediums auf. Genauer gesagt wird das gasförmige Medium erwärmt, bevor es in den Verdampfer eintritt.

Die vorliegende Erfindung ist weiterhin auf ein Verfahren zum Sterilisieren von Behältnissen gerichtet, wobei in einen Verdampfer ein gasförmiges Medium eingeführt wird und mittels einer Einspritzeinrichtung eine Substanz eingespritzt wird, welche Wasserstoffperoxid (H₂O₂) enthält und wobei diese Substanz an einer Heizeinrichtung verdampft wird und über eine zweite in dem Gehäuse angeordnete Öffnung das entstehende Gemisch aus dem gasförmigen Medium und der verdampften Substanz abgeführt wird. Erfindungsgemäß erstreckt sich die Längsrichtung der Einspritzeinrichtung senkrecht zu einer Ebene der Heizeinrichtung, auf welcher das Wasserstoffperoxid auftrifft, und das gasförmige Medium verläuft innerhalb eines Rohrabschnitts wenigstens abschnittsweise parallel zu der Längsrichtung zu der Einspritzeinrichtung, wobei die Einspritzeinrichtung wenigstens abschnittsweise innerhalb des Rohrabschnitts verläuft.

Bei einem weiteren möglichen Verfahren erstreckt sich die Längsrichtung der Einspritzeinrichtung senkrecht zu einer Ebene der Heizeinrichtung und das gasförmige Medium verläuft innerhalb eines Rohrabschnitts wenigstens abschnittsweise parallel zu der Längsrichtung zu der Einspritzeinrichtung, wobei sich der Rohrabschnitt wenigstens abschnittsweise innerhalb des Gehäuses erstreckt.

Auch hier handelt es sich bei der Substanz vorzugsweise um Wasserstoffperoxid. Im Rahmen des Verfahrens wird durch die wenigstens abschnittsweise parallele Führung der Einspritzeinrichtung in eine besonders günstige Strömungsführung der austretenden Substanz erreicht. Auch kann eine strömungsgünstige Abführung des Gemisches aus dem Verdampfer erreicht werden.

Darin zeigen:
- Fig. 1: eine perspektivische Ansicht eines erfindungsgemäßen Verdampfers;
- Fig. 2: eine Querschnittsdarstellung des Verdampfers aus Fig. 1;
- Fig. 3: eine Draufsicht auf einen erfindungsgemäßen Verdampfer; und
- Fig. 4: einen Verdampfer in einer weiteren Ausführungsform; und
- Fig. 5: eine Dichtungseinrichtung für den Verdampfer aus Fig. 4.

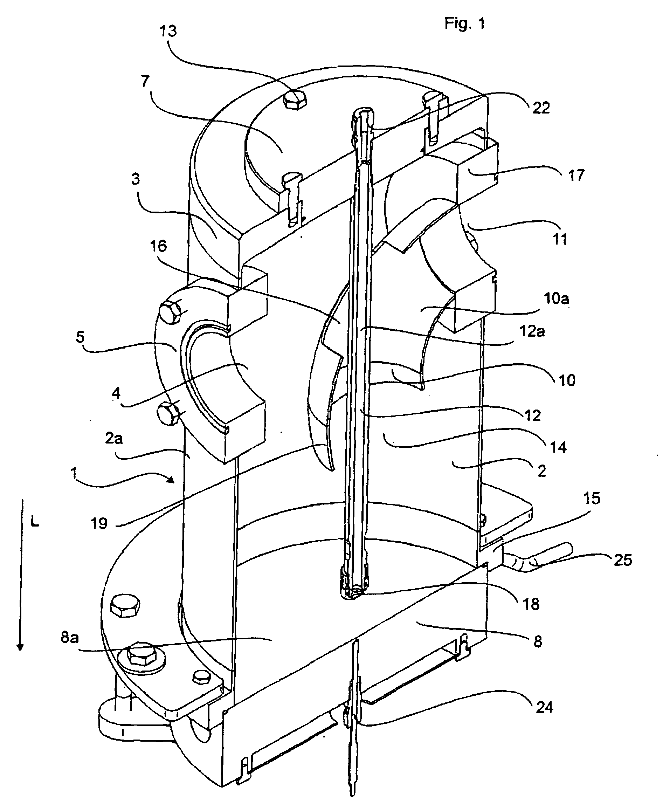

Fig. 1 zeigt einen erfindungsgemäßen Verdampfer 1. Dieser Verdampfer weist ein Gehäuse 2 mit einer umlaufenden Wandung 2a und einem Deckelabschnitt 3 auf, der durch einen mit Schrauben 13 aufgeschraubten Deckel 7 verschlossen ist. Nach unten hin ist das Gehäuse durch eine Heizeinrichtung 8 bzw. eine Heizfläche 8a abgeschlossen. Die Ebene dieser Heizfläche steht senkrecht zur Figurenebene. Das Bezugszeichen 12 bezieht sich auf eine Einspritzeinrichtung, deren Längsrichtung sich senkrecht zu der Ebene der Heizfläche 8a erstreckt. Durch diese Einspritzeinrichtung 12, genauer gesagt einen Kanal 12a im Innenraum der Einspritzeinrichtung 12 wird Wasserstoffperoxid in das Gehäuse 2 und in Richtung der Heizeinrichtung 8 geführt. Bei der in Fig. 1 gezeigten Ausführungsform ist die Einspritzeinrichtung 12 konzentrisch mit der Umfangswand 2a angeordnet. Am unteren Ende weist die Einspritzeinrichtung 12 eine Düse 18 auf, über die Wasserstoffperoxid austreten kann und auf die Heizfläche 8a gespritzt wird.

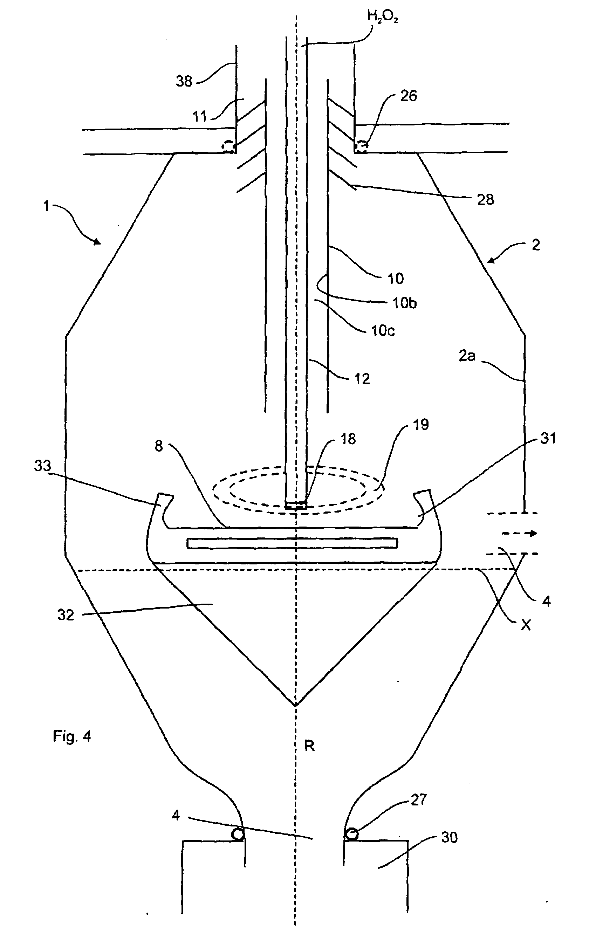

Das Bezugszeichen 24 bezieht sich auf einen Temperatursensor zum Ermitteln der Temperatur der Heizeinrichtung 8. Oberhalb der Heizeinrichtung 8 ist ein Flansch 15 zur Befestigung des Gehäuses 2 auf der Heizeinrichtung 8 vorgesehen. Dieser Flansch 15 ist bevorzugt, ebenso wie das Gehäuse 2, aus Edelstahl gefertigt.'In einer bevorzugten Aufführungsform ist die Heizeinrichtung 8 aus Aluminium gefertigt und weist an ihren Rändern Seitenwände auf, die nicht geradlinig verlaufen sondern eine nach außen weisende Ausnehmung aufweisen. Durch eine derartige Ausnehmung kann die Luftströmung in der Umgebung der Heizeinrichtung 8 verbessert werden und damit eine verbesserte Verdampfung des Wasserstoffperoxids erreicht werden. Diese Ausgestaltung der Seitenwände ist in Fig. 4 dargestellt.

Oberhalb der Heizeinrichtung 8 ist ein Trägerring 15 vorgesehen, auf dem wiederum das Gehäuse 2 abgestützt ist. Bevorzugt ist dieser Trägerring 15 aus einem Material hergestellt, welches einen geringen Wärmeleistungsquozienten aufweist, damit sich über die von der Heizeinrichtung 8 abgegebene Wärme das Gehäuse 2 nicht oder nicht wesentlich erwärmt.

Das Bezugszeichen 11 bezieht sich auf eine erste Öffnung (Eintrittsöffnung) in dem Gehäuse 2, durch welche hindurch das gasförmige Medium in das Innere des Gehäuses 2 gelangen kann. Die erste Öffnung 11 ist dabei in einem Flansch 17 angeordnet, der an der Gehäusewandung 2a befestigt ist. Im Inneren des Gehäuses 2 schließt sich an die erste Öffnung 11 ein Rohrabschnitt 10 an. Dieser Rohrabschnitt 10 weist einen gekrümmten Bereich 10a und eine erste Austrittsöffnung 14, durch welche hindurch das gasförmige Medium austreten kann, auf. Damit erfolgt die Strömungsführung der erwärmten Luft abschnittsweise parallel zu der Wasserstoffperoxidzufuhr über die Einspritzeinrichtung 12. Zu diesem Zweck wird die durch die erste Öffnung 12 einströmende Luft in den gekrümmten Abschnitt 10a umgelenkt.

Das Bezugszeichen 16 bezieht sich auf eine zweite Austrittsöffnung in dem Rohrabschnitt 10. Durch diese Öffnung tritt einerseits die Einspritzeinrichtung 12 hindurch. Auf diese Weise kann die Einspritzeinrichtung wenigstens teilweise innerhalb des Rohrabschnitts 10 geführt werden. Daneben kann durch die zweite Austrittsöffnung 6 auch ein Anteil des gasförmigen Mediums aus dem Rohrabschnitt austreten und in Richtung einer Austrittsöffnung 4 gelangen. Über die Austrittsöffnung 4, die ebenfalls mittels eines Flansches 5 an der Gehäusewandung 2a angeordnet ist, kann das in dem Verdampfer entstehende Gasgemisch abgeführt und anschließend den zu sterilisierenden Behältnissen zugeführt werden.

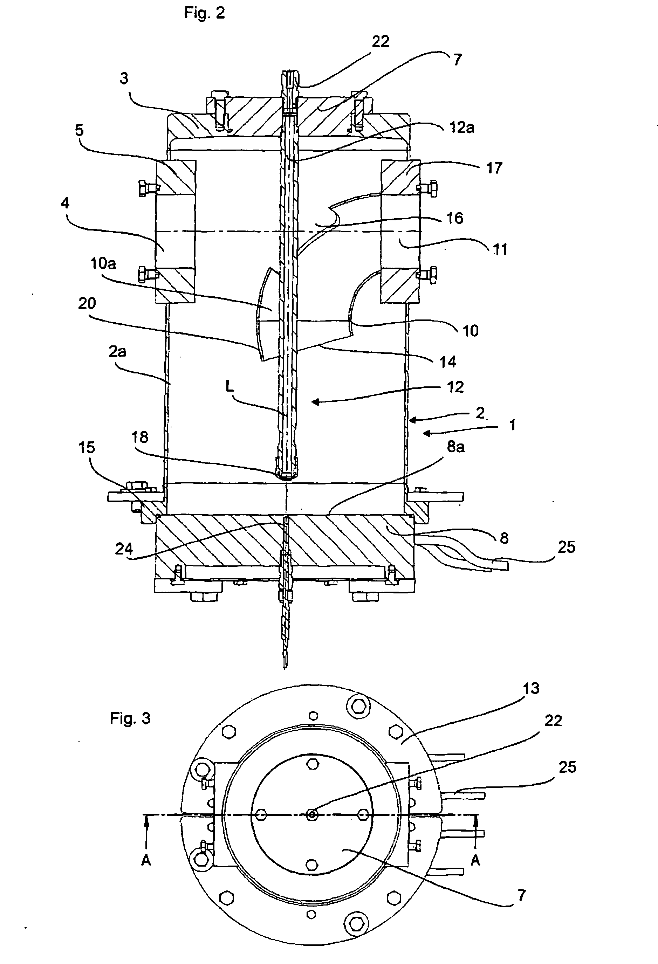

Fig. 2 zeigt eine Querschnittsdarstellung eines erfindungsgemäßen Verdampfers. Man erkennt, dass die Düse 18 der Einspritzeinrichtung 12 wesentlich weiter in das Innere des Gehäuses hineinragt bzw. wesentlich näher an der Heizeinrichtung 8 angeordnet ist als die erste Austrittsöffnung 14 des Rohrabschnitts 10. In dem Endabschnitt 20 des Rohrabschnitts 10 wird das hindurch tretende gasförmige Medium nicht genau in der Längsrichtung geführt sondern in einem gewissen Winkel hierzu geneigt. Auf diese Weise wird erreicht, dass durch entsprechende Reflexionen des gasförmigen Mediums an der Heizeinrichtung eine besonders vorteilhafte Durchmischung mit dem verdampfenden Wasserstoffperoxid auftritt. Genauer gesagt wird das durch den Rohrabschnitt 10 hindurch tretende gasförmige Medium wieder geringfügig zurück in Richtung der ersten Öffnung 11 geführt. Das Bezugszeichen 22 bezieht sich auf einen Anschluß für eine Wasserstoffperoxidzuleitung. Das Bezugszeichen 25 bezieht sich auf eine Zuleitung für ein Heizmedium für die Heizeinrichtung 8.

Man erkennt, dass die zweite Austrittsöffnung 16 in dem Rohrabschnitt 10 derart ausgelegt ist, dass sich ein größerer Abschnitt dieser zweiten Austrittsöffnung 16 rechts bezüglich der Einspritzeinrichtung 12 das heißt der Einspritzlanze 12 befindet und nur ein kleiner Abschnitt links hiervon. Auf diese Weise kann der Rohrabschnitt innerhalb des Gehäuses und damit auch seine Oberfläche gering gehalten werden.

Fig. 3 zeigt eine Draufsicht auf die in Fig. 2 gezeigte Ausführungsform. Man erkennt, dass der Anschluß 22 und auch die gesamte Einspritzeinrichtung 12 im Wesentlichen konzentrisch bezüglich des Deckels 7 und auch bezüglich des Gehäuses 2 gehalten sind.

Fig. 4 zeigt schematisch zwei weitere Ausführungsform eines erfindungsgemäßen Verdampfers. Dabei ist eine dieser beiden weiteren Ausführungsformen durch gestrichelte Linien verdeutlicht. Bei diesen Ausführungsformen ist die erste Öffnung 11 nicht in einer Seitenwand des Gehäuses 2 vorgesehen sondern in dem oberen Abschnitt bzw. Deckel. Auf diese Weise ist es möglich, auch den Rohrabschnitt 10 vollständig konzentrisch bezüglich der Einspritzeinrichtung 12 zu führen, ohne einen gekrümmten Abschnitt vorsehen zu müssen. Genauer gesagt ist im Inneren des Gehäuses kein Bogen des Rohrabschnitts vorgesehen, aber ein eventueller Bogen ist außerhalb des Gehäuses 2 angeordnet. Der Vorteil dieser Ausführungsform besteht einerseits darin, dass innerhalb des Verdampfers bzw. des Gehäuses 2 weniger Oberfläche vorhanden ist. Weiterhin ist es durch die im Wesentlichen symmetrische Gestaltung der in Fig. 4 gezeigten Ausführungsform möglich, ein strömungsgünstiges Design des gesamten Verdampfers herzustellen.

Die Strömungsführung erfolgt direkt von der ersten Öffnung 11 hin zu der zweiten Öffnung 4, welche in dem Boden des Gehäuses 2 bzw. unterhalb der Heizeinrichtung 8 vorgesehen ist. In dem Strömungspfad zwischen der ersten Öffnung 11 und der zweiten Öffnung 4 ist auch hier die Heizeinrichtung 8 vorgesehen, die zum Verdampfen des Wasserstoffperoxids dient.

Die Heizeinrichtung 8 weist bei dieser Ausführungsform an den Seitenrändern Ausnehmungen 31 auf, die, wie oben erläutert eine verbesserte Luftströmung bewirken.

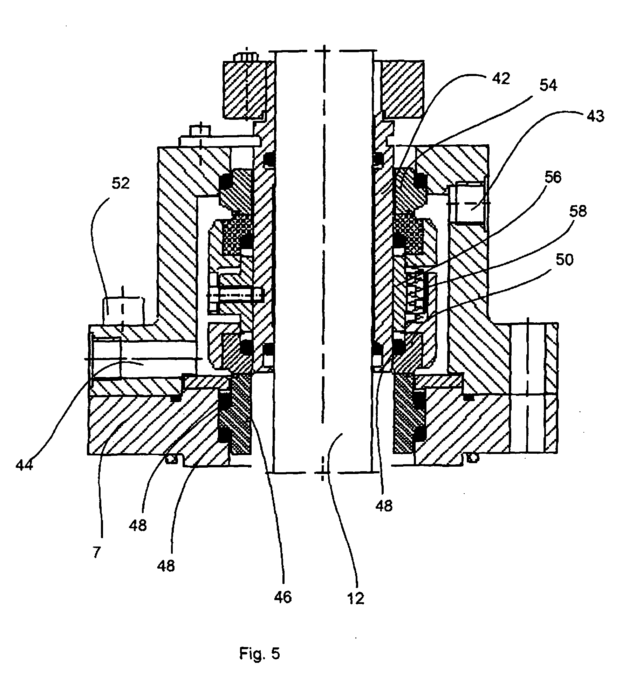

Bei dieser Ausführungsform steht das Gehäuse 2 mit der Einspritzeinrichtung 10, 11, 12 und die untere Öffnung 4 dreht. Der Vorteil dieser Ausführungsform besteht darin, dass die Heizeinrichtung 8 nicht über Schleifringträger versorgt werden muss. Eine für eine derartige Ausführungsform geeignete Dichtungseinrichtung 27 wird unten genauer in Fig. 5 erläutert.

Das Bezugszeichen 32 bezieht sich auf einen Träger für die Heizeinrichtung, der einen schräg nach innen ragenden Abschnitt 33 aufweist, der ebenfalls die Strömung des verdampften Wasserstoffperoxids begünstigt.

Bei der weiteren in Fig. 4 gezeigten Ausführungsform ist die zweite Öffnung 4 in der Seitenwand 2a vorgesehen. Dieser Sachverhalt ist in Fig. 4 durch die gestrichelten Linien dargestellt. Genauer gesagt ist eine Vielzahl von zweiten Öffnungen 4 in der Seitenwand 2a angeordnet, wobei bevorzugt diese zweiten Öffnungen 4 gleichmäßig in der Umfangsrichtung des Gehäuses 2 vorgesehen sind. Ausgehend von diesen Öffnungen werden die einzelnen Behältnisse mit dem entstehenden Gemisch versorgt.

Bei dieser (durch die gestrichelten Linien dargestellten) Ausführungsform dreht das Gehäuse 2 und die Einspritzeinrichtung 10, 11, 12 ruht. Der Vorteil dieser Ausführungsform besteht darin, dass die Heizeinrichtung gleichmäßig mit Wasserstoffperoxid beaufschlagt wird und die Dichtung funktional integriert wird. Daher ist bei dieser Ausführungsform lediglich die obere Dichtungseinrichtung 26 vorgesehen. Das Gehäuse endet bei dieser Ausführungsform direkt unterhalb der Heizeinrichtung 8, wie durch die gestrichelte Linie X angedeutet.

Das eingeführte gasförmige Medium wird einerseits in einem Zwischenraum 10c zwischen der Einspritzeinrichtung 12 und der Wandung 10b des Rohrabschnitts 10 geführt und andererseits auch außerhalb des Rohrabschnitts 10. Zu diesem Zweck sind Ablenkeinrichtungen 28 vorgesehen, welche das einströmende gasförmige Medium schräg und radial nach außen in Richtung der Wandung 2a des Gehäuses ablenken. Auch hierdurch werden die Strömungsverhältnisse im Inneren des Verdampfers 1 verbessert.

Ein weiterer Vorteil der in Fig. 4 gezeigten zweiten Ausführungsform besteht darin, dass diese Ausführungsform besonders günstig auf einem Behandlungskarusell angebracht werden kann. Während bei den Verdampfern im Stand der Technik eine Vielzahl von Verdampfern nötig ist, wobei die einzelnen Verdampfer jeweils ein oder zwei Behältnisse sterilisieren, kann die in Fig. 4 gezeigte Ausführungsform eine Vielzahl von Behältnissen desinfizieren. Genauer gesagt kann die in Fig. 4 gezeigte Anordnung teilweise drehend um die Rotationsachse R angeordnet sein.

Mit anderen Worten ist zur Adaption des in Fig. 4 gezeigten Verdampfers auf ein Behandlungskarusell die Trennung in einen stehenden Teil und in einen drehenden Teil in dem Übergangsbereich zwischen einem Außenrohr 38 und dem Verdampfer 1 gut möglich. Bei der in Fig. 4 gezeigten Ausführungsform steht die Einspritzeinrichtung 12 und das Außenrohr 38 fest und das Gehäuse 2 einschließlich der Heizeinrichtung 8 dreht sich. Auf diese Weise kann auf eine spezielle Dichtung für die Zuführung des Wasserstoffperoxids verzichtet werden, da die entsprechenden Teile stehen. Weiterhin ist, wie erwähnt, ein zentraler Aufbau für die gesamte Luftaufbereitung möglich. Die Abdichtung des Gehäuses erfolgt im Gasbereich, wobei eventuell gesperrte Dichtungen das heißt Dichtungen, die ein Dichtungsgas bzw. Dichtungsmedium einsetzen, verwendet werden können. Das Bezugszeichen 26 zeigt grob schematisch entsprechende Dichtungen. Die Abdichtung erfolgt hier im Gegensatz zu den anderen Ausführungsformen gegenüber dem entstehenden Gasgemisch.

Das Bezugszeichen 18 bezieht sich auch hier auf eine Düse zum Einspritzen des Wasserstoffperoxids. Anstelle oder neben dieser Düse kann jedoch auch eine (gestrichelt dargestellte) Ringdüse 19 verwendet werden. Diese Ringdüse 19 weist an ihrer Unterseite Öffnungen auf, aus denen das Wasserstoffperoxid nach unten d.h. in Richtung der Heizeinrichtung 8 austreten kann. Vorzugsweise tritt das Wasserstoffperoxid durch diese Düse 19 senkrecht nach unten aus. Es ist jedoch auch möglich, die einzelnen unterschiedlichen Ausführungsformen in Fig. 4 miteinander zu kombinieren, beispielsweise die Ringdüse 19 mit den in der Seitenwand angeordneten Öffnungen 4.

Fig. 5 zeigt eine Dichtungseinrichtung 26 für einen Verdampfer nach Figur 4. Diese Dichtungseinrichtung 26 ist derart gestaltet, dass die Einspritzeinrichtung 12 steht und sich das Gehäuse bzw. der mit dem Gehäuse verbundene Deckel 7 dreht. Genauer wird hier eine Gleitringdichtung 26 mit zwei Gleitringen 50, 54 verwendet. Das Bezugszeichen 46 bezieht sich auf einen Gegenring, der zum Abstützen des unteren Gleitrings 50 dient. Dieser Gegenring ist mit zwei O-Ringen 48 gegenüber dem Deckel 7 abgedichtet.

An der Einspritzeinrichtung 12 ist eine Hülse 42 angeordnet und zwischen der Einspritzeinrichtung 12 und der Hülse 42 zwei weitere O-Ringe 48. Zwischen den beiden Gleitringen 50, 54 ist ein Stellring 56 vorgesehen, sowie mehrere Federn (von denen nur eine Feder 58 dargestellt ist) welche die beiden Gleitringe 50, 54 belasten. Dieser Stellring 56 ist fest an der Hülse 42 angeordnet. Das Bezugszeichen 43 bezieht sich auf einen Einlass für ein Sperrmedium und das Bezugszeichen 44 auf einen Auslass für dasselbe. Dieses Sperrmedium, bei dem es sich besonders bevorzugt um sterilisiertes Wasser oder Luft handelt, dient dazu, um einen ungewollten Austritt von Wasserstoffperoxid oder auch von Wasserstoffperoxid - Gemisch aus dem Verdampfer zu verhindern. Daneben kann dieses Sperrmedium auch als Gleitmedium eingesetzt werden.

## Patentansprüche

1. Verdampfer (1) zum Sterilisieren von Behältnissen, welcher ein Gehäuse (2) aufweist, mit einer ersten in dem Gehäuse (2) angeordneten Öffnung (11), um dem Gehäuse (2) ein gasförmiges Medium zuzuführen, mit einer Einspritzeinrichtung (12), um in das Gehäuse (2) eine Substanz einzuspritzen, welche Wasserstoffperoxid (H₂O₂) enthält, mit einer innerhalb des Gehäuses (2) angeordneten Heizeinrichtung (8) zum Verdampfen des Wasserstoffperoxids und mit einer zweiten in dem Gehäuse (2) angeordneten Öffnung (4), um ein Gemisch aus dem gasförmigen Medium und dem verdampften Wasserstoffperoxid aus dem Gehäuse (2) abzuführen, **dadurch gekennzeichnet, dass** sich die Längsrichtung (L) der Einspritzeinrichtung (12) senkrecht zu einer Ebene der Heizeinrichtung (8), auf welcher das Wasserstoffperoxid auftrifft, erstreckt und sich an die erste Öffnung (11) ein Rohrabschnitt (10) anschließt, der sich wenigstens abschnittsweise in einer Längsrichtung (L) der Einspritzeinrichtung (12) erstreckt, wobei die Einspritzeinrichtung (12) wenigstens abschnittsweise innerhalb des Rohrabschnitts (10) verläuft.

2. Verdampfer (1) nach wenigstens einem der vorangegangenen Ansprüche, **dadurch gekennzeichnet, dass** sich der Rohrabschnitt (10) innerhalb des Gehäuses (2) wenigstens abschnittsweise in der Längsrichtung (L) der Einspritzeinrichtung (12) erstreckt.

3. Verdampfer (1) nach wenigstens einem der vorangegangenen Ansprüche, **dadurch gekennzeichnet, dass** der Rohrabschnitt (10) eine erste Austrittsöffnung (14) aufweist, die derart angeordnet ist, dass das gasförmige Medium in einer zu der Längsrichtung (L) parallelen Richtung aus dem Rohrabschnitt (10) austritt.

4. Verdampfer (1) nach wenigstens einem der vorangegangenen Ansprüche, **dadurch gekennzeichnet, dass** der Rohrabschnitt (10) eine zweite Austrittsöffnung (16) aufweist, die zwischen der ersten in dem Gehäuse angeordneten Öffnung (11) und der ersten Austrittsöffnung (14) vorgesehen ist.

5. Verdampfer (1) nach wenigstens einem der vorangegangenen Ansprüche, **dadurch gekennzeichnet, dass** der Rohrabschnitt (10) einen gekrümmten Bereich (10a) aufweist.

6. Verdampfer (1) nach wenigstens einem der vorangegangenen Ansprüche, **dadurch gekennzeichnet, dass** die Einspritzeinrichtung (12) eine Austrittsdüse (18) für das Wasserstoffperoxid aufweist und diese Austrittsdüse (18) unterhalb der ersten Austrittsöffnung (14) des Rohrabschnitts (10) angeordnet ist.

7. Verdampfer (1) nach wenigstens einem der vorangegangenen Ansprüche, **dadurch gekennzeichnet, dass** die erste Öffnung (11) in einem Deckelabschnitt (3) des Gehäuses (2) angeordnet ist.

8. Verdampfer (1) nach wenigstens einem der vorangegangenen Ansprüche, **dadurch gekennzeichnet, dass** die Einspritzeinrichtung (12) und der Rohrabschnitt (10) wenigstens abschnittsweise im Wesentlichen konzentrisch zueinander sind.

9. Verdampfer (1) nach wenigstens einem der vorangegangenen Ansprüche, **dadurch gekennzeichnet, dass** die zweite Öffnung (4) in einem Bodenabschnitt des Gehäuses (2) angeordnet ist.

10. Verdampfer (1) nach wenigstens einem der vorangegangenen Ansprüche, **dadurch gekennzeichnet, dass** der gesamte Rohrabschnitt (10) konzentrisch zu der Einspritzeinrichtung (12) angeordnet ist.

11. Verdampfer (1) nach wenigstens einem der vorangegangenen Ansprüche, **dadurch gekennzeichnet, dass** die Einspritzeinrichtung (12) drehbar gegenüber dem Gehäuse (2) angeordnet ist.

12. Verdampfer (1) nach wenigstens einem der vorangegangenen Ansprüche, **dadurch gekennzeichnet, dass** der Rohrabschnitt (10) drehbar gegenüber dem Gehäuse (2) angeordnet ist.

13. Verfahren zum Sterilisieren von Behältnissen, wobei in einen Verdampfer (1) ein gasförmiges Medium eingeführt wird und mittels einer Einspritzeinrichtung (12) eine Substanz eingespritzt wird, welche Wasserstoffperoxid (H₂O₂) enthält und wobei diese Substanz an einer Heizeinrichtung (8) verdampft wird und über eine zweite in dem Gehäuse (2) angeordnete Öffnung (4) das entstehende Gemisch aus dem gasförmigen Medium und er verdampften Substanz abgeführt wird, **dadurch gekennzeichnet, dass** sich die Längsrichtung (L) der Einspritzeinrichtung (12) senkrecht zu einer Ebene der Heizeinrichtung (8), auf welcher das Wasserstoffperoxid auftrifft, erstreckt und das gasförmige Medium innerhalb eines Rohrabschnittes (10) wenigstens abschnittsweise parallel zu der Längsrichtung (L) der Einspritzeinrichtung (12) geführt wird, wobei die Einspritzeinrichtung (12) wenigstens abschnittsweise innerhalb des Rohrabschnitts (10) verläuft.

## Claims

1. Vaporizer (1) for the sterilization of containers, comprising a housing (2), a first opening (11) arranged in the housing (2) for supplying a gaseous medium to the housing (2), an injection device (12) for injecting into the housing (2) a substance which contains hydrogen peroxide (H₂O₂), a heating device (8) arranged inside the housing (2) for vaporizing the hydrogen peroxide, and a second opening (4) arranged in the housing (2) for discharging a mixture of the gaseous medium and the vaporized hydrogen peroxide from the housing (2), **characterized in that** the longitudinal direction (L) of the injection device (12) extends perpendicular to a plane of the heating device (8) onto which the hydrogen peroxide impinges and the first opening (11) is adjoined by a pipe section (10) which extends at least partially in a longitudinal direction (L) of the injection device (12), wherein the injection device (12) runs at least partially inside the pipe section (10).

2. Vaporizer (1) according to at least one of the preceding claims, **characterized in that** the pipe section (10) inside the housing (2) extends at least partially in the longitudinal direction (L) of the injection device (12).

3. Vaporizer (1) according to at least one of the preceding claims, **characterized in that** the pipe section (10) has a first exit opening (14) which is arranged in such a way that the gaseous medium exits from the pipe section (10) in a direction parallel to the longitudinal direction (L).

4. Vaporizer (1) according to at least one of the preceding claims, characterize in that the pipe section (10) has a second exit opening (16) which is provided between the first opening (11) arranged in the housing and the first exit opening (14).

5. Vaporizer (1) according to at least one of the preceding claims, **characterized in that** the pipe section (10) has a curved region (10a).

6. Vaporizer (1) according to at least one of the preceding claims, **characterized in that** the injection device (12) has an outlet nozzle (18) for the hydrogen peroxide and this outlet nozzle (18) is arranged below the first exit opening (14) of the pipe section (10).

7. Vaporizer (1) according to at least one of the preceding claims, **characterized in that** the first opening (11) is arranged in a lid section (3) of the housing (2).

8. Vaporizer (1) according to at least one of the preceding claims, **characterized in that** the injection device (12) and the pipe section (10) are at least partially essentially concentric with one another.

9. Vaporizer (1) according to at least one of the preceding claims, **characterized in that** the second opening (4) is arranged in a base section of the housing (2).

10. Vaporizer (1) according to at least one of the preceding claims, **characterized in that** the entire pipe section (10) is arranged concentric with the injection device (12).

11. Vaporizer (1) according to at least one of the preceding claims, **characterized in that** the injection device (12) is arranged such that it can rotate relative to the housing (2).

12. Vaporizer (1) according to at least one of the preceding claims, **characterized in that** the pipe section (10) is arranged such that it can rotate relative to the housing (2).

13. Method for the sterilization of containers, wherein a gaseous medium is introduced into a vaporizer (1) and a substance which contains hydrogen peroxide (H₂O₂) is injected by means of an injection device (12), and wherein this substance is vaporized at a heating device (8) and the resulting mixture of the gaseous medium and the vaporized substance is discharged via a second opening (4) arranged in the housing (2), **characterized in that** the longitudinal direction (L) of the injection device (12) extends perpendicular to a plane of the heating device (8) onto which the hydrogen peroxide impinges and that the gaseous medium is guided within a pipe section (10) at least partially parallel to the longitudinal direction (L) of the injection device (12), wherein the injection device (12) runs at least partially inside the pipe section (10).

## Revendications

1. Évaporateur (1) pour la stérilisation de récipients, lequel comporte un carter (2), comportant un premier orifice (11) agencé dans le carter (2) pour acheminer un fluide gazeux vers l'intérieur du carter (2), comportant un dispositif d'injection (12) pour injecter dans le carter (2) une substance contenant du peroxyde d'hydrogène (H₂O₂), comportant un dispositif de chauffage (8) agencé à l'intérieur du carter (2) pour transformer en vapeur le peroxyde d'hydrogène, et comportant un deuxième orifice (4) agencé dans le carter (2) pour évacuer hors du carter (2) un mélange constitué du fluide gazeux et du peroxyde d'hydrogène transformé en vapeur, **caractérisé en ce que** le sens longitudinal (L) du dispositif d'injection (12) est orienté perpendiculairement à un plan du dispositif de chauffage (8), sur lequel parvient le peroxyde d'hydrogène, et un tronçon de tube (10) est adjacent au premier orifice (11) et s'étend au moins dans certaines zones dans un sens longitudinal (L) du dispositif d'injection (12), ledit dispositif d'injection (12) s'étendant au moins dans certaines zones à l'intérieur du tronçon de tube (10).

2. Évaporateur (1) selon la revendication précédente, **caractérisé en ce que** le tronçon de tube (10) s'étend à l'intérieur du carter (2) au moins dans certaines zones dans le sens longitudinal (L) du dispositif d'injection (12).

3. Évaporateur (1) selon au moins l'une quelconque des revendications précédentes, **caractérisé en ce que** le tronçon de tube (10) comporte un premier orifice de sortie (14) agencé de telle sorte que le fluide gazeux sort du tronçon de tube (10) dans une direction parallèle au sens longitudinal (L).

4. Évaporateur (1) selon au moins l'une quelconque des revendications précédentes, **caractérisé en ce que** le tronçon de tube (10) comporte un deuxième orifice de sortie (16) prévu entre le premier orifice (11) agencé dans le carter et le premier orifice de sortie (14).

5. Évaporateur (1) selon au moins l'une quelconque des revendications précédentes, **caractérisé en ce que** le tronçon de tube (10) comporte une zone coudée (10a).

6. Évaporateur (1) selon au moins l'une quelconque des revendications précédentes, **caractérisé en ce que** le dispositif d'injection (12) comporte une buse de sortie (18) pour le peroxyde d'hydrogène et ladite buse de sortie (18) est agencée en dessous du premier orifice de sortie (14) du tronçon de tube (10).

7. Évaporateur (1) selon au moins l'une quelconque des revendications précédentes, **caractérisé en ce que** le premier orifice (11) est agencé dans une partie formant couvercle (3) du carter (2).

8. Évaporateur (1) selon au moins l'une quelconque des revendications précédentes, **caractérisé en ce que** le dispositif d'injection (12) et le tronçon de tube (10) sont sensiblement concentriques l'un à l'autre au moins dans certaines zones.

9. Évaporateur (1) selon au moins l'une quelconque des revendications précédentes, **caractérisé en ce que** le deuxième orifice (4) est agencé dans une partie formant le fond du carter (2).

10. Évaporateur (1) selon au moins l'une quelconque des revendications précédentes, **caractérisé en ce que** la totalité du tronçon de tube (10) est disposée concentriquement au dispositif d'injection (12).

11. Évaporateur (1) selon au moins l'une quelconque des revendications précédentes, **caractérisé en ce que** le dispositif d'injection (12) est monté rotatif par rapport au carter (2).

12. Évaporateur (1) selon au moins l'une quelconque des revendications précédentes, **caractérisé en ce que** le tronçon de tube (10) est monté rotatif par rapport au carter (2).

13. Procédé pour la stérilisation de récipients, selon lequel un fluide gazeux est introduit dans un évaporateur (1) et une substance contenant du peroxyde d'hydrogène (H₂O₂) est injectée au moyen d'un dispositif d'injection (12), et selon lequel ladite substance est transformée en vapeur au niveau d'un dispositif de chauffage (8) et le mélange formé, constitué du fluide
gazeux et de la substance transformée en vapeur, est évacué via un deuxième orifice (4) agencé dans le carter (2), **caractérisé en ce que** le sens longitudinal (L) du dispositif d'injection (12) est orienté perpendiculairement à un plan du dispositif de chauffage (8), sur lequel parvient le peroxyde d'hydrogène, et le fluide gazeux est guidé à l'intérieur d'un tronçon de tube (10) au moins dans certaines zones parallèlement au sens longitudinal (L) du dispositif d'injection (12), ledit dispositif d'injection (12) s'étendant au moins dans certaines zones à l'intérieur du tronçon de tube (10).
